# EUROPÄISCHE PATENTSCHRIFT

(11) **EP 1 085 858 B1**
(45) Veröffentlichungstag und Bekanntmachung des Hinweises auf die Patenterteilung: **27.10.2004**
(21) Anmeldenummer: 99922167.4
(22) Anmeldetag: 04.05.1999
(51) Int. Cl.: A61K 9/70

(54) **TRANSDERMALES THERAPEUTISCHES SYSTEM ZUR ANWENDUNG VON CANDESARTAN**
TRANSDERMAL THERAPEUTIC SYSTEM FOR THE ADMINISTRATION OF CANDESARTAN
SYSTEME THERAPEUTIQUE TRANSDERMIQUE POUR ADMINISTRATION DE CANDESARTAN

(30) Priorität: 06.05.1998 DE 19820151
(43) Veröffentlichungstag der Anmeldung: 28.03.2001
(73) Patentinhaber: Hexal AG, 83607 Holzkirchen (DE)
(72) Erfinder: STRÜNGMANN, Thomas, D-83607 Holzkirchen (DE)
(86) Internationale Anmeldenummer: PCT/EP1999/003029
(87) Internationale Veröffentlichungsnummer: WO 1999/056734

(56) Entgegenhaltungen:
- EP-A- 0 612 523
- EP-A- 0 752 249

## Beschreibung

Die Erfindung betrifft ein wirkstoffhaltiges transdermales System zur Anwendung von Candesartan und/oder dessen pharmazeutisch unbedenklichen Estern und/ oder Salzen.

Candesartan (2-Ethoxy-1-[[2'-(1H-tetrazol-5-yl)biphenyl-4-yl]methyl]-benzimidazol-7-carbonsäure) ist ein hoch spezifischer, nicht peptidischer Angiotensin-II-Rezeptor-Antagonist. Es besitzt eine hohe Spezifität und eine starke Affinität an den AT₁-Rezeptor sowie eine lange Bindungsdauer und somit eine lange Wirksamkeit. Candesartan wird hauptsächlich zur Behandlung von essentieller Hypertonie (nicht organbedingter Bluthochdruck), Herzerkrankungen, Schlaganfällen, Nephritis (EP-0459136 B1) und linksventrikulärer Hypertrophie verwendet.

Bei der oralen Verabreichung wird der Ester (Candesartancilexetil) von Candesartan und 1-(Cyclohexyloxycarbonyloxy)-ethanol als "Prodrug" verwendet (EP-0459136 B1), um die für die Magenpassage nötige Stabilität zu gewährleisten und somit die Bioverfügbarkeit zu erhöhen (Kubo, K.; Kohara, Y. and co-workers; J. of Medicinal Chemistry; 36(16)2343-2349/1993). Dieser Ester wird im Gastrointestinaltrakt durch Esterhydrolyse vollständig in seine Wirkform Candesartan überführt, welches um 30% aktiver ist als der Ester. Candesartan wird dann weitläufig im Gewebe und in den Blutgefäßen verteilt. Die Eliminierung von Candesartan aus den Blutgefäßwänden erfolgt wesentlich langsamer als aus dem Plasma, wodurch die langanhaltende Wirkung hervorgerufen wird . Candesartan wird teilweise in der Leber zu inaktiven Metaboliten weiter metabolisiert. Candesartan und seine Metaboliten werden dann nach der Leber- Gallen- Passage über den Kot und Urin ausgeschieden. Die im Darm abgespaltene Esterseitenkette vom Candesartancilexetil wird hauptsächlich als Cyclohexanol absorbiert und im Gewebe verteilt. In der Leber findet dann der Abbau zu Cyclohexandiol, Cyclohexantriol und anderen Abbauprodukten statt. Die Bioverfügbarkeit von Candesartan beträgt dabei nur 14%. Der maximale therapeutische Effekt wird bei oraler Einnahme nach 4 Wochen erreicht, da durch das langsame Besetzen der Rezeptoren eine allmähliche Blutdrucksenkung erfolgt.

Bisher erfolgt die Verabreichung von Candesartancilexetil ausschließlich oral oder intravenös. Da Candesartan während der Magenpassage durch die Magensäure abgebaut wird, muß der Wirkstoff entweder verestert oder eine aufwendige Darreichungsform wie z.B. eine magensaftresistente Beschichtung geschaffen werden. Dadurch entstehen zusätzliche Kosten, sowohl für die Maschinen und Arbeitskräfte als auch für das zusätzlich benötigte Material.
Bei der oralen Applikation von Wirkstoffen ist die Bioverfügbarkeit häufig unbefriedigend. In diesem Fall beträgt sie nur 14%. Die hepatische Metabolisierung des Wirkstoffes bei der ersten Leberpassage kann zu unerwünschten Konzentrationsverhältnissen und toxischen Nebenprodukten sowie zur Verminderung der Wirkung führen.

Aufgabe der vorliegenden Erfindung ist es nun, ein transdermales System für die systemische Verabreichung von Candesartan und/ oder einem seiner pharmazeutisch unbedenklichen Ester oder Salze bereitzustellen, wobei die Nachteile bisher angewandter oraler oder intravenöser Verabreichungsformen vermieden werden sollen.

Es wurde nun überraschenderweise gefunden, daß Candesartan und/ oder dessen pharmazeutisch unbedenkliche Ester und Salze mittels eines transdermalen therapeutischen Systems in der Art und Weise verabreicht werden kann, daß ein therapeutisch wirksamer Blutspiegel erzielt wird. Durch die Möglichkeit der Verwendung des Wirkstoffes Candesartan und/ oder dessen pharmazeutisch unbedenklicher Ester und Salze, die direkt systemisch zur Wirkung gelangen, kann die Bioverfügbarkeit beträchtlich erhöht und die Höhe der Dosierung stark gesenkt werden. Die Belastung des Organismus und die Beeinträchtigung der Leber durch die Metabolisierung kann somit wesentlich verringert werden. Durch die Verwendung eines transdermalen therapeutischen Systems wird eine gesteuerte , kontrollierte Wirkstoffabgabe ermöglicht, so daß hohe Blutplasmaschwankungen vermieden und ein konstanter Blutplasmaspiegel auch über mehrere Tage hinweg garantiert werden kann. Die optimale Wirkung des Wirkstoffes wird so bequem und zuverlässig erreicht. Der maximale therapeutische Effekt wird schon nach 3 Wochen erreicht.
Ebenfalls als Vorteil ist die einfache und bequeme Anwendung von Pflastern im Vergleich zur oralen oder intravenösen Darreichung zu sehen. Da das System extern appliziert wird, kann es ohne Wechsel sehr lange auf diese Weise seine ihm zugedachte Funktion erfüllen. Dies ist mit oralen Systemen schlechterdings unmöglich, da sie durch die Verdauungstätigkeit nach längstens einem Tag den Organismus verlassen. Zudem ist es für den Patienten einfacher und angenehmer anstatt 1 Mal pro Tag eine Tablette einnehmen zu müssen, nur 1-2 Mal pro Woche an die Medikamenteneinnahme denken zu müssen.

Die der Erfindung zugrundeliegende Aufgabe wird nun durch ein transdermales therapeutisches System mit einem Gehalt an Candesartan und/ oder einem seiner pharmazeutisch unbedenklichen Ester oder Salze gelöst, insbesondere durch Candesartan und/oder Candesartancilexetil.

Als mögliche Salze von Candesartan kommen vor allem Alkalisalze, wie z.B. das Kalium-, Natrium- und Lithiumsalz sowie das Ammoniumsalz in Frage.

Candesartan und/ oder einer seiner pharmazeutisch verträglichen Ester oder Salze als Wirkstoff kann femer in Kombination mit weiteren bekannten Wirkstoffen vor allem Diuretika und Ca- Antagonisten angewendet werden, z.B. Hydrochlorothiazid (HCTZ) oder Amlodipin. Diese Wirkstoffe bewirken einen additiven antihypertensiven Effekt.

Das erfindungsgemäße transdermale therapeutische System kann ein Pflaster darstellen. Bei diesem Pflaster kann es sich um ein Matrix- oder Membransystem handeln, welches eine undurchlässige Deckschicht und eine abziehbare Schutzschicht aufweist. Als Bestandteil der undurchlässigen Deckschicht kommen Polyester, Polypropylen, Polyurethan oder Polyethylen in Frage, die bei Bedarf metallisiert oder pigmentiert werden können. Für die abziehbare Schutzschicht kommen Polyester, Polypropylen, Polysiloxan, Polyacrylat, Ethylenvinylacetat, Polyurethan, Polyisobuten oder Papier mit Silikon- und /oder Polyethylenbeschichtung in Betracht.

Das Matrixpflaster kann aus einer undurchlässigen Deckschicht, aus einer oder mehreren den Wirkstoff und/ oder einer seiner pharmazeutisch unbedenklichen Ester oder Salze und gegebenenfalls weiteren Wirkstoffen und/ oder Permeationsförderer und/ oder Aminosäuren enthaltenden, selbstklebenden Matrixschicht oder einer Matrixschicht, die mit einem Haftkleber beschichtet ist und einer abziehbaren Schutzschicht bestehen. Bei dem in der Matrix enthaltenen Wirkstoff kann es sich um Candesartan und/ oder um dessen pharmazeutisch unbedenklichen Ester oder Salze, sowie bei der Kombination zudem um weitere Wirkstoffe wie Ca- Antagonisten oder Diuretika z.B. Amlodipin oder HCTZ handeln.

Für die Matrix können die medizinisch üblichen Matrixbildner wie Polyacrylat, Silikon, Polyisobutylen, Kautschuk, kautschukähnliche synthetische Homo-, Co- oder Blockpolymere, Butylkautschuk, Styrol/ Isopren- Copolymerisat, Polyurethane, Copolymere des Ethylens, Polysiloxane oder Styrol/ Butadien- Copolymerisat verwendet werden.

Eine weitere Ausführungsform der Erfindung stellt ein Membransystem dar. Dieses kann aus einer undurchlässigen Deckschicht, einem wirkstoffhaltigen Reservoir oder einer Reservoirschicht, einer semipermeablen Membran, einer fakultativen Haftklebeschicht und einer abziehbaren Schutzschicht bestehen. Das Reservoir kann Candesartan und/ oder einen seiner pharmazeutisch unbedenklichen Ester oder Salze, gegebenenfalls weitere Wirkstoffe und/ oder Permeationsförderer, Stabilisatoren, Emulgatoren, Verdickungsmittel und/ oder übliche Membransystem- bzw. Reservoirpflaster- Hilfsmittel enthalten. Das Reservoir bzw. die Reservoirschicht liegt zwischen der Deckschicht und der Membran. Als Gelbildner können bei Bedarf Methylcellulose, Hydroxypropylcellulose, Hydroxyethylcellulose, Carboxyvinylpolymer, Natrium- Plyoxilat, Carboxymethylcellulose oder ein Gemisch aus diesen verwendet werden.

Die Membran, die üblicherweise aus inerten Polymeren, insbesondere auf Basis von Polypropylen, Polyvinylacetat, Polyamid, Ethylen- Vinylacetat- Copolymeren oder Silikon, besteht, kann je nach Porengröße eine die Wirkstoffreisetzung kontrollierende Wirkung haben.

Für die Haftklebeschicht des oben beschriebenen erfindungsgemäßen Matrix- oder Membransystems kann man ein druckempfindliches Klebemittel beispielsweise auf Polyurethanbasis, Polyisobutylenbasis, Polyvinyletherbasis, Siliconbasis oder Acrylatbasis wählen.

Bei dem Klebemittel auf Silkonbasis kann es sich um Silikonkleber handeln, welche auf zwei Hauptbestandteilen basieren: Ein Polymer oder Klebstoff, insbesondere Polysiloxan, und ein tackerhöhendes Harz. Der Polysiloxankleber ist gewöhnlich mit einem Vemetzer für den Kleber, typischerweise mit einem hochmolekularen Polydiorganosiloxan, und mit dem Harz zubereitet, um über ein angemessenes organisches Lösungsmittel eine dreidimensionale Silikatstruktur zu ergeben. Die Zumischung des Harzes zu Polymer ist der wichtigste Faktor, um die physikalischen Eigenschaften der polysiloxanen Kleber zu ändern; vgl. beispielsweise Sobieski, et al., "Silicone Pressure Sensitive Adhesives", Handbook of Pressure Sensitive Adhesive Technology, 2^{nd} ed., pp. 508-517 (D. Satas, ed.), Van Nostrand Reinhold, New sive Technology, 2^{nd} ed., pp. 508-517 (D. Satas, ed.), Van Nostrand Reinhold, New York (1989).

Ein weiteres Beispiel für ein druckempfindliches Klebemittel auf Silikonbasis ist trimethyliertes Siliciumdioxid, das mit Polydimethylsiloxan mit endständigen Trimethylsiloxy- Gruppen behandelt worden ist.

Bei den Klebemitteln auf Acrylatbasis kann es sich um ein beliebiges Homopolymer, Copolymer oder Terpolymer, bestehend aus verschiedenen Acrylsäurederivaten handeln.

So können die Acrylatpolymere Polymere eines oder mehrerer Monomere von Acrylsäuren und anderen copolymerisierbaren Monomeren sein. Außerdem können die Acrylatpolymere Copolymere von Alkylacrylaten und/ oder -methacrylaten und/ oder copolymerisierbaren sekundären Monomeren oder Monomeren mit funktionellen Gruppen umfassen. Verändert man den Betrag jeder Sorte, die als Monomer hinzugefügt ist, können die kohäsiven Eigenschaften der daraus resultierenden Acrylatpolymere verändert werden. Im allgemeinen besteht das Acrylatpolymer aus mindestens 50 Gew.-% eines Acrylat-, Methacrylat-, Alkylacrylat- oder Alkylmethacrylat-Monomers, 0 bis 20 % eines funktionellen Monomers, copolymerisierbar mit Acrylat, und 0 bis 40 % eines anderen Monomeren.

Im folgenden sind Acrylatmonomere aufgeführt, die mit Acrylsäure, Methacrylsäure, Butylacrylat, Butylmethacrylat, Hexylacrylat, Hexylmethacrylat, Isooctylacrylat, Isooctylmethacrylat, 2-Ethylhexylacrylat, 2-Ethylhexylmethacrylat, Decylacrylat, Decylmethacrylat, Dodecylacrylat, Dodecylmethacrylat, Tridecylacrylat und Tridecylmethacrylat verwendet werden können.

So können funktionelle Monomere, die mit den oben genannten Acrylaten, Methacrylaten, Alkylacrylaten oder -methacrylaten copolymerisierbar sind, eingesetzt werden, beispielsweise Acrylsäure, Methacrylsäure, Maleinsäure, Maleinanhydrid, Hydroxyethylacrylat, Hydroxypropylacrylat, Acrylamid, Dimethylacrylamid, Acrylnitril, Dimethylaminoethylacrylat, Dimethylaminoethylmethacrylat, tert.-Butylaminoethylacrylat, ter.-Butylaminoethylmethacrylat, Methoxyethylacrylat und Methoxyethylmethacrylat.

Weiter Einzelheiten und Beispiele für druckempfindliche Acrylate, welche für die Erfindung geeignet sind, sind in Satas Handbook of Pressure Sensitive Adhesive Technology "Acrylic Adhesives", 2^{nd} ed., pp. 396-456 (D. Satas, ed.), Van Nostrand Reinhold, New York (1989) beschrieben.

Als Permeationsförderer lassen sich ein- und/oder mehrwertige aliphatische, cycloaliphatische und /oder aromatisch- aliphatische Alkohole mit jeweils bis zu acht C- Atomen, z.B. Ethanol, 1,2-Propandiol, Dexpanthenol und/ oder Polyethylenglykol; Alkohol/ Wasser- Gemische; gesättigte und/ oder ungesättigte Fettalkohole mit jeweils 8- 18 C- Atomen; Terpene; z.B. Cineol, Carveol, Menthon, Terpineol, Verbenon, Menthol, Limonen, Thymol, Cymen, Terpinen-4-ol, Neomenthol, Geraniol, Fenchon; Gemische aus Terpenen und Ethanol und/ oder Propylenglykol; Teebaumöl; gesättigte und/ oder ungesättigte cyclische Ketone; Alkyl-Methylsulfoxide; gesättigte und/ oder ungesättigte Fettsäuren mit jeweils 8- 18 C- Atomen; deren Ester und Salze; natürliches Vitamin E; synthetisches Vitamin E und/ oder Vitamin E-Derivate; Sorbitanfettsäureester und ethoxylierte Sorbitanfettsäureester; Azone (Laurocapram); Azone gemischt mit Alkoholen; Harnstoff; 1-Alkylpyrrolidon; Blockcopolymere von Polyethylenglykol und Dimethylsiloxan mit kationischer Gruppe an einem Ende; Folat-Polyethylenglykol-Liposom, Proliposom; Polyoxyethylen-10-stearylether; Gemisch aus Polyoxyethylen-10-stearylether und Glyceryldilaurat; Dodecyl-2-(N,N-dimethylamino)-propanoltetradecanoat und/ oder Dodecyl-2-(N,N-dimethylamino)-propianat; N-Acetylprolinatester mit mehr als 8 C-Atomen; nichtionische Tenside, z.B. Laurylether, Ester von Polyoxyethylen; Ethosom (Phospholipidvesikel); Dimethyl(arylimino)sulfuran; Gemisch aus Ölsäureanaloga und Propylenglykol; Gemisch aus Padimat O, Oktylsalicylat, Oktylmethoxycinnimat und Laurocapram und/ oder Gemische aus all diesen Komponenten verwenden.

Die Erfindung wird durch nachstehende Beispiele näher erläutert, ohne aber den Erfindungsumfang damit einzuschränken.

### Beispiel 1 (Matrix- Pflaster)

Es werden 11,1 g Candesartancilexetil in 75 g Aceton reinst gelöst und mit 8 g Copherol F1300 versetzt. Die klare Lösung wird zu 169 g eines ca. 36%igen Acrylat- Copolymeren (Duro- Tak 387-2353, Nat. Starch & Chemical B.V.) gegeben und gerührt. Die homogene Lösung wird auf einer silikonisierten Polyesterfolie (z.B. 75 µm) oder auf silikonisiertem Papier ausgestrichen und bei 35 °C bis 85 °C getrocknet, so daß ein Matrix- Trockengewicht von 80 ± 10% g/m² erhalten wird. Auf die Matrixseite wird anschließend die abziehbare Schutzschicht (z.B. Polyester 15 µm) kaschiert.
Es werden TTS mit einer Fläche von 20 cm² ausgestanzt.
Ein Pflaster dieser Größe enthält 16 mg Candesartan und 16 mg α-Tocopherol.

### Beispiel 2 (Reservoir- Pflaster) (siehe Zeichnung)

Zuerst werden 138,4 g Candesartancilexetil in 861,6 g einer Mischung aus Ethanol abs. 65% (V/G), Copherol F1300 10% (V/G) und Hydroxypropylcellulose 1% (V/G) unter Rühren gelöst. Diese Mischung stellt die Verumlösung für das Reservoir dar. Mit 400 ± 5% mg der Verumlösung wird das Reservoir gefüllt.
Das transdermale therapeutische System besteht zum einen aus der fakultativen Klebeschicht, die den Klebering bildet. Auf diese Schicht wird eine heißsiegelfähige, undurchlässige Deckschicht aufgebracht. Auf der der Haut zugewandten Seite wird das Reservoir auf der Deckschicht befestigt und mit einer mikroporösen EVA-Membran (Cotran 9702, 3M) abgeschlossen. Als abziehbare Schutzschicht dient eine silikonisierte PET- Folie.

### Ein Pflaster enthält also:

| | |
|---|---|
| Candesartancilexetil | 55,36 mg (entsprechend 40 mg Candesartan) |
| Copherol F1300 | 40 mg |
| Ethanol abs. | 300,64 mg |
| Hydroxypropylcellulose | 4 mg |

## Patentansprüche

1. Transdermales therapeutisches System mit einem Gehalt an Candesartan oder einem seiner pharmazeutisch unbedenklichen Ester oder Salze.

2. Transdermales therapeutisches System nach Anspruch 1, **gekennzeichnet durch** Candesartan als Wirkstoff.

3. Transdermales therapeutisches System nach Anspruch 1, **gekennzeichnet durch** Candesartancilexetil als Wirkstoff.

4. Transdermales therapeutisches System nach Anspruch 1, **gekennzeichnet durch** die Ammonium- und/oder Alkalisalze von Candesartan als Wirkstoff.

5. Transdermales therapeutisches System nach einem der vorangegangenen Ansprüche, **gekennzeichnet durch** Candesartan oder eines seiner pharmazeutisch verträglichen Ester oder Salze als Wirkstoff in Kombination mit weiteren Wirkstoffen.

6. Transdermales therapeutisches System nach Anspruch 5, **gekennzeichnet durch** mindestens einen weiteren Wirkstoff, der die Candesartan- Wirkung verstärkt.

7. Transdermales therapeutisches System nach Anspruch 5 oder 6, **gekennzeichnet durch** Diuretika und/ oder Ca-Antagonisten als weitere Wirkstoffe.

8. Transdermales therapeutisches System nach einem der vorhergehenden Ansprüche in Form eines Pflasters mit einer undurchlässigen Deckschicht und einer abziehbaren Schutzschicht, insbesondere in Form eines Matrixsystems oder eines Membransystems.

9. Transdermales therapeutisches System nach Anspruch 8, **gekennzeichnet durch** eine Deckschicht auf Basis von Polyester, Polypropylen, Polyurethan oder Polyethylen, gegebenenfalls jeweils metallisiert oder pigmentiert.

10. Transdermales therapeutisches System nach Anspruch 8, **gekennzeichnet durch** eine abziehbare Schutzschicht auf Basis von Polyester, Polypropylen, Polysiloxan, Polyacrylat, Ethylenvinylacetat, Polyurethan, Polyisobuten oder Papier mit Silikon- und /oder Polyethylenbeschichtung.

11. Transdermales therapeutisches System nach Anspruch 8, 9 oder 10, **dadurch gekennzeichnet, daß** es sich um ein Matrixsystem mit
• einer undurchlässigen Deckschicht,
• einer oder mehreren wirkstoffhaltigen selbstklebenden Matrixschicht(en) oder einer oder mehreren wirkstoffhaltigen Matrixschicht(en), die mit einem Haftkleber beschichtet sind,
• einer abziehbaren Schutzschicht und
• Candesartan oder einem seiner pharmazeutisch verträglichen Ester oder Salze als Wirkstoff handelt.

12. Transdermales therapeutisches System nach Anspruch 11, **gekennzeichnet durch** eine Matrixschicht auf Basis von Polyacrylat, Silikon, Polyisobutylen, Kautschuk, kautschukähnliche synthetische Homo-, Co- oder Blockpolymere, Butylkautschuk, Styrol/Isopren- Copolymerisat, Polyurethane, Copolymere des Ethylens, Polysiloxane oder Styrol/ Butadien- Copolymerisat.

13. Transdermales therapeutisches System nach Anspruch 8, 9 oder 10, **dadurch gekennzeichnet, daß** es sich um ein Membransystem mit
• einer undurchlässigen Deckschicht,
• einem wirkstoffhaltigen Reservoir oder einer wirkstoffhaltigen Reservoirschicht,
• einer mikroporösen oder semipermeablen Membran,
• einer fakultativen Haftklebeschicht,
• Candesartan oder einem seiner pharmazeutisch verträglichen Ester oder Salze als Wirkstoff handelt.

14. Transdermales therapeutisches System nach Anspruch 13, **gekennzeichnet durch** eine Membran auf Basis eines inerten Polymeren, insbesondere Polypropylen, Polyvinylacetat, Polyamid, Ethylen- Vinylacetat- Copolymeren oder Silikon.

15. Transdermales therapeutisches System nach einem der vorhergehenden Ansprüche, **gekennzeichnet durch** einen Permeationsförderer, insbesondere ein- und/oder mehrwertige aliphatische, cycloaliphatische und /oder aromatisch- aliphatische Alkohole mit jeweils bis zu acht C- Atomen und/ oder Polyethylenglykol; Alkohol/ Wasser- Gemische; gesättigte und/ oder ungesättigte Fettalkohole mit jeweils 8- 18 C- Atomen; Terpene; Gemische aus Terpenen und Ethanol und/ oder Propylenglykol; Teebaumöl; gesättigte und/ oder ungesättigte cyclische Ketone; Alkyl- Methylsulfoxide; gesättigte und/ oder ungesättigte Fettsäuren mit jeweils 8- 18 C- Atomen; deren Ester und Salze; natürliches Vitamin E; synthetisches Vitamin E und/ oder Vitamin E- Derivate; Sorbitanfettsäureester und ethoxylierte Sorbitanfettsäureester; Azone (Laurocapram); Azone gemischt mit Alkoholen; Harnstoff; 1-Alkylpyrrolidon; Blockcopolymere von Polyethylenglykol und Dimethylsiloxan mit kationischer Gruppe an einem Ende; Folat-Polyethylenglykol-Liposom, Proliposom; Polyoxyethylen-10-stearylether; Gemisch aus Polyoxyethylen-10-stearylether und Glyceryldilaurat; Dodecyl-2-(N,N-dimethylamino)-propanoltetradecanoat und/ oder Dodecyl-2-(N,N-dimethylamino)-propianat; N-Acetylprolinatester mit mehr als 8 C-Atomen; nichtionische Tenside, Ester von Polyoxyethylen; Ethosom (Phospholipidvesikel); Dimethyl(arylimino)sulfuran; Gemisch aus Ölsäureanaloga und Propylenglykol; Gemisch aus Padimat O, Oktylsalicylat, Oktylmethoxycinnimat und Laurocapram und/ oder Gemische aus all diesen Komponenten

## Claims

1. A transdermal therapeutic system with a content of candesartan or one of its pharmaceutically suitable esters or salts.

2. A transdermal therapeutic system according to claim 1, **characterized by** candesartan as active ingredient.

3. A transdermal therapeutic system according to claim 1, **characterized by** candesartan cilexetil as active ingredient.

4. A transdermal therapeutic system according to claim 1, **characterized by** the ammonium and/or alkali metal salts of candesartan as active ingredient.

5. A transdermal therapeutic system according to any of the preceding claims, **characterized by** candesartan or one of its pharmaceutically acceptable esters or salts as active ingredient in combination with other active ingredients.

6. A transdermal therapeutic system according to claim 5, **characterized by** at least one other active ingredient which enhances the effect of candesartan.

7. A transdermal therapeutic system according to claim 5 or 6, **characterized by** diuretics and/or Ca channel blockers as other active ingredients.

8. A transdermal therapeutic system according to any of the preceding claims in the form of a plaster with an impermeable covering layer and a detachable protective layer, in particular in the form of a matrix system or of a membrane system.

9. A transdermal therapeutic system according to claim 8, **characterized by** a covering layer based on polyester, polypropylene, polyurethane or polyethylene, where appropriate in each case metalized or pigmented.

10. A transdermal therapeutic system according to claim 8, **characterized by** a detachable protective layer based on polyester, polypropylene, polysiloxane, polyacrylate, ethylene/vinyl acetate, polyurethane, polyisobutene or paper with silicone and/or polyethylene coating.

11. A transdermal therapeutic system according to claim 8, 9 or 10, **characterized in that** it is a matrix system with
• an impermeable covering layer,
• one or more active ingredient-containing contact adhesive matrix layer(s) or one or more active ingredient-containing matrix layer(s) coated with a contact adhesive,
• a detachable protective layer and
• candesartan or one of its pharmaceutically acceptable esters or salts as active ingredient.

12. A transdermal therapeutic system according to claim 11, **characterized by** a matrix layer based on polyacrylate, silicone, polyisobutylene, rubber, rubber-like synthetic homo-, co- or block polymers, butyl rubber, styrene/isoprene copolymer, polyurethanes, copolymers of ethylene, polysiloxanes or styrene/butadiene copolymer.

13. A transdermal therapeutic system according to claim 8, 9 or 10, **characterized in that** it is a membrane system with
• an impermeable covering layer,
• an active ingredient-containing reservoir or an active ingredient-containing reservoir layer,
• a microporous or semipermeable membrane,
• an optional contact adhesive layer,
• candesartan or one of its pharmaceutically acceptable esters or salts as active ingredient.

14. A transdermal therapeutic system according to claim 13, **characterized by** a membrane based on an inert polymer, in particular polypropylene, polyvinyl acetate, polyamide, ethylene/vinyl acetate copolymer or silicone.

15. A transdermal therapeutic system according to any of the preceding claims, **characterized by** a permeation promoter, in particular monohydric and/or polyhydric aliphatic, cycloaliphatic and/or aromatic-aliphatic alcohols each with up to 8 C atoms, and/or polyethylene glycol; alcohol/water mixtures; saturated and/or unsaturated fatty alcohols each with 8-18 C atoms; terpenes; mixtures of terpenes and ethanol and/or propylene glycol; tea tree oil; saturated and/or unsaturated cyclic ketones; alkyl methyl sulfoxides; saturated and/or unsaturated fatty acids each with 8-18 C atoms; the esters and salts thereof; natural vitamin E; synthetic vitamin E and/or vitamin E derivatives; sorbitan fatty acid esters and ethoxylated sorbitan fatty acid esters; Azone (laurocapram); Azone mixed with alcohols; urea; 1-alkylpyrrolidone; block copolymers of polyethylene glycol and dimethylsiloxane with cationic groups at one end; folate-polyethylene glycol liposome, proliposome; polyoxyethylene 10 stearyl ether; mixture of polyoxyethylene 10 stearyl ether and glyceryl dilaurate; dodecyl 2-(N,N-dimethylamino)propanoltetradecanoate and/or dodecyl 2-(N,N-dimethylamino)propionate; N-acetylprolinate esters with more than 8 C atoms; nonionic surfactants, esters of polyoxyethylene; ethosome (phospholipid vesicle); dimethyl(arylimino)sulfurane; mixture of oleic acid analogs and propylene glycol; mixture of padimate O, octyl salicylate, octyl methoxycinnamate and laurocapram and/or mixtures of all these components.

## Revendications

1. Système thérapeutique transdermique avec une teneur de Candesartan ou de l'un de ses esters ou sels pharmaceutiques ne présentant pas de risque pour la santé.

2. Système thérapeutique transdermique selon la revendication 1, **caractérisé par** Candesartan comme principe actif.

3. Système thérapeutique transdermique selon la revendication 1, **caractérisé par** Candesartan cilexetil comme principe actif.

4. Système thérapeutique transdermique selon la revendication 1, **caractérisé par** les sels d'ammonium et/ou sels alcalins de Candesartan comme principe actif.

5. Système thérapeutique transdermique selon l'une des revendications précédentes, **caractérisé par** Candesartan ou l'un de ses esters ou sels pharmaceutiquement compatibles comme principe actif en combinaison avec d'autres principes actifs.

6. Système thérapeutique transdermique selon la revendication 5, **caractérisé par** au moins un autre principe actif renforçant l'effet de Candesartan.

7. Système thérapeutique transdermique selon la revendication 5 ou 6, **caractérisé par** des diurétiques et/ou inhibiteurs calciques comme autre principe actif.

8. Système thérapeutique transdermique selon l'une des revendications précédentes, sous forme d'un tibre transdermique avec une couche superficielle imperméable et une couche de protection retirable, surtout sous la forme d'un système de matrices ou d'un système de membranes.

9. Système thérapeutique transdermique selon la revendication 8, **caractérisé par** une couche supérieure fabriquée sur la base de polyester, polypropylène, polyurethane ou polyéthylène, le cas échéant respectivement métallisé ou pigmenté.

10. Système thérapeutique transdermique selon la revendication 8, **caractérisé par** une couche retirable fabriquée sur la base de polyester, polypropylène, polysiloxane, polyacrylate, acétate de vinyle éthylène, polyuréthane, polyisobutène ou sur la base de papier avec un revêtement en silicone et/ou polyéthylène.

11. Système thérapeutique transdermique selon la revendication 8, 9 ou 10 **caractérisé par le fait qu'**il s'agisse d'un système de matrices constitué
• d'une couche superficielle imperméable,
• d'une ou plusieurs couche(s) de matrices autocollante(s) contenant le principe actif ou d'une ou plusieurs couche(s) de matrices contenant le principe actif revêtue(s) d'une colle de contact,
• d'une couche de protection retirable et
• de Candesartan ou de l'un de ses esters ou sels pharmaceutiquement compatibles comme principe actif.

12. Système thérapeutique transdermique selon la revendication 11 **caractérisé par** une couche de matrice fabriquée sur la base de polyacrylate, silicone, polyisobutylène, caoutchouc, monopolymères, copolymères ou polymères à blocs synthétiques caoutchouteux, caoutchouc butyl, copolymère styrène-isoprène, polyuréthane, copolymères de l'éthylène, polysiloxane ou copolymère styrène-butadienne.

13. Système thérapeutique transdermique selon la revendication 8, 9 ou 10 **caractérisé par le fait qu'**il s'agisse d'un système de membranes constitué
• d'une couche superficielle imperméable,
• d'un réservoir contenant le principe actif ou d'une couche réservoir contenant le principe actif,
• d'une membrane microporeuse ou semi-perméable
• d'une couche de colle de contact facultative,
• de Candesartan ou de l'un de ses esters ou sels pharmaceutiquement compatibles comme principe actif.

14. Système thérapeutique transdermique selon la revendication 13 **caractérisé par** une membrane fabriquée sur la base d'un polymère inerte, tel que polypropylène, acétate de polyvinyle, polyamide, copolymères d'acétate de vinyle éthylène ou silicone.

15. Système thérapeutique transdermique selon l'une des revendications précédentes, **caractérisé par** un activateur de pénétration, tels que les alcools aliphatiques, cycloaliphatique et/ou aromatiques univalents et/ou polyvalents avec respectivement au moins 8 atomes de C et/ou de polyéthylène glycol ; mélanges alcool/eau ; alcools gras saturés et/ou insaturés avec chacun 8-18 atomes de C ; terpènes, mélanges de terpènes et d'éthanol et/ou de propylène glycol ; huile d'arbre à thé ; cétones cycliques saturés et/ou insaturés ; methylalkylsulfoxydes ; acides gras saturés et insaturés avec respectivement 8 - 18 atomes de C, leurs esters et sels ; vitamine E naturelle ; vitamine E synthétique et/ou dérivés de la vitamine E ; ester d'acides gras de sorbitane et ester d'acides gras de sorbitane éthoxylé ; Azones (Laurocapram), Azones mélangés avec des alcools ; urée ; 1-akylpyrrolydone, polymères à blocs de polyéthylène glycol et diméthylsiloxane avec un groupe cationique situé en bout de chaîne liposome de folate et de polyethylène-glycol, proliposomes ; polyoxyéthylène-10-stéaryl éther ; mélanges de polyoxyéthylène-10-stéaryl éther et dilaurate de glycéryle ; dodécyl-2-(N, N-diméthylamino)-propanol tetradécanoate; dodécyl-2-(N, N-diméthylamino)-propianate ; N-ester d'acétylprolinate avec plus de 8 atomes de C ; tensides anioniques ; ester de polyoxyéthylène; Ethosom (vésicule phospholipidique); diméthyl(arylimino)sulfurane; mélanges d'analogues d'acide oléique et de propylène-glycol; mélanges de padimate-O ; octyl salicylate ; octyl méthoxycinnamate et Laurocapran et/ou mélanges de tous ces composants.
